# EUROPÄISCHE PATENTSCHRIFT

(11) **EP 2 748 125 B1**
(45) Veröffentlichungstag und Bekanntmachung des Hinweises auf die Patenterteilung: **03.12.2014**
(21) Anmeldenummer: 12750385.2
(22) Anmeldetag: 23.08.2012
(51) Int. Cl.: C07B 43/04

(54) **VERFAHREN ZUR KONTINUIERLICHEN NUKLEOPHILEN ADDITION AN AKTIVIERTEN KOHLENSTOFF-KOHLENSTOFF-MEHRFACHBINDUNGEN**
METHOD FOR CONTINUOUS NUCLEOPHILIC ADDITION TO ACTIVATED MULTIPLE CARBON-CARBON BONDS
PROCÉDÉ D'AJOUT NUCLÉOPHILE CONTINU SUR DES COMPOSÉS MULTIPLES DE CARBONE-CARBONE ACTIFS

(30) Priorität: 23.08.2011 EP 11178446
(43) Veröffentlichungstag der Anmeldung: 02.07.2014
(73) Patentinhaber: BYK-Chemie GmbH, 46483 Wesel (DE)
(72) Erfinder: ISSBERNER, Jörg, 47877 Willich-Neersen (DE); NAGELSDIEK, René, 46499 Hamminkeln (DE); TLAUKA, Frank, 46147 Oberhausen (DE); GÖBELT, Bernd, 46487 Wesel (DE); BECKMANN, Tom, 46499 Hamminkeln (DE)
(74) Vertreter: Leifert & Steffan
(86) Internationale Anmeldenummer: PCT/EP2012/066426
(87) Internationale Veröffentlichungsnummer: WO 2013/026903

(56) Entgegenhaltungen:
- ARNO BEHR AND JULIA LESCHINSKI: "Application of the solvent water in two-phase telomerisation reactions and recycling of the homogeneous palladium catalysts", GREEN CHEMISTRY, ROYAL SOCIETY OF CHEMISTRY, CAMBRIDGE, GB, Bd. 11, Nr. 5, 1. Mai 2009 (2009-05-01), Seiten 609-613, XP008145024, ISSN: 1463-9262, DOI: 10.1039/B820305A [gefunden am 2009-02-17]

## Beschreibung

Die Erfindung betrifft ein Verfahren zur kontinuierlichen Addition von Nukleophilen an Verbindungen mit aktivierten Kohlenstoff-Kohlenstoff-Mehrfachbindungen. Derartige Reaktionen werden in der Literatur auch als Michael-Additionen bzw. Michael-Reaktionen bezeichnet.

Bei einer Michael-Reaktion handelt es sich im engeren Sinne um eine Reaktion zwischen einem Carbanion als nukleophilem Reaktanten und einer aktivierten Kohlenstoff-Kohlenstoff-Doppelbindung, d.h. einer mit mindestens einer elektronenziehenden Gruppe substituierten Kohlenstoff-Kohlenstoff-Doppelbindung als elektrophilem Akzeptor unter Neuknüpfung einer Kohlenstoff-Kohlenstoff-Einfachbindung. In der vorliegenden Erfindung wird ein weiter gefasster Begriff der Michael-Reaktion bzw. Michael-Addition zugrunde gelegt. Hierbei kann der nukleophile Reaktant anstelle eines Carbanions beispielsweise auch ein Amin oder Thiol sein, wobei es zur Neuknüpfung einer Stickstoff-Kohlenstoff- bzw. SchwefelKohlenstoff-Bindung kommt. Der elektrophile Akzeptor ist im Sinne dieser Erfindung nicht auf eine aktivierte Kohlenstoff-Kohlenstoff-Doppelbindung beschränkt, es kann sich vielmehr auch um eine aktivierte Kohlenstoff-Kohlenstoff-Dreifachbindung handeln, wobei das Additionsprodukt dann wiederum eine Kohlenstoff-Kohlenstoff-Doppelbindung enthält. In jedem Fall erfolgt die kovalente Bindungsknüpfung zwischen einem Donor (Nukleophil, "Michael-Donor") und einem aktivierten, elektrophilen Alken oder Alkin ("Michael-Akzeptor"). Die Begriffe des engeren und weiteren Verständnisses einer Michael-Reaktion sind weit verbreitet und beispielsweise auch im Band 2 des Lehrbuchs für Lacke und Beschichtungen des Autors H. Kittel (S. Hirzel Verlag Stuttgart/Leipzig 1998, 2. Auflage, Kapitel 2.2.3.3 "Reaktivsysteme auf Basis der Michael-Reaktion", Seiten 328 bis 334), in Eur. J. Org. Chem. 2010, 2009-2016 oder in Prog. Polymer Sci. 2006, 31, 487-531 exemplifiziert.

Da bei der Umsetzung einer Kohlenstoff-Kohlenstoff-Doppelbindung mit einem Michael-Donor aus der Doppelbindung eine Einfachbindung bzw. bei der Umsetzung einer Kohlenstoff-Kohlenstoff-Dreifachbindung mit einem Michael-Donor aus der Dreifachbindung eine Doppelbindung entsteht, ist die betreffende Additionsreaktion stark exotherm, was bei der verfahrenstechnischen Durchführung das Problem der Wärmeabfuhr mit sich bringt. Gleichzeitig stellt die Exothermie auch ein Sicherheitsrisiko dar, insbesondere, wenn man bedenkt, dass als nicht umgesetzter Reaktionspartner alkenisch oder alkinisch ungesättigte Verbindungen in hoher Konzentration vorliegen, welche das Risiko mit sich bringen, sich im Rahmen einer Radikalkettenreaktion unter Umständen schlagartig umzusetzen. Zusätzlich zum Sicherheitsrisiko beeinträchtigen solche Nebenreaktionen erfahrungsgemäß auch die Qualität des entstandenen Produkts (z.B., indem Moleküle eines deutlich höheren Molgewichts, gebildet werden).

Für eine Anwendbarkeit betreffender Additionsprodukte ist es jedoch besonders wichtig, qualitativ hochwertige Produkte zu erhalten, die weitgehend frei von unerwünschten Nebenprodukten sind. Desweiteren ist es wünschenswert, die bei der Additionsreaktion freiwerdende Wärmemenge in einem sicheren Verfahren möglichst effektiv abzuführen, so dass eine ungewollte Polymerisation ("Autopolymerisation") der alkenisch oder alkinisch ungesättigten Komponente, welche nicht nur eine Veränderung der Produkteigenschaften nach sich zieht, sondern v.a. ein hohes Risiko für die Anlagensicherheit mit sich bringt, ausgeschlossen werden kann. Eine solche Wärmeabfuhr kann nach klassischen Verfahren z.B. durch Arbeiten in verdünnter Lösung erfolgen, wodurch entsprechende Mengen eines Lösemittels eingeschleppt werden. Da diese Lösungsmittel bei vielen Anwendungen jedoch unerwünscht sind (viele Lösemittel sind als Gefahrstoffe eingestuft und in vielen Anwendungen sind flüchtige organische Verbindungen, sogenannte "Volatile Organic Compounds" (VOC), nicht akzeptabel), müssen sie in diesem Fall durch aufwendige Verfahren wieder vollständig abgetrennt werden. Die Abtrennung ist mit Zeitaufwand und Betriebskosten verbunden, zudem werden die hierbei anfallenden Mengen des abgetrennten Lösemittels in vielen Fällen nicht weiter verwendet, sondern müssen entsorgt werden, sind damit also verlorene Ressourcen. Zudem können die zum Abtrennen des Lösungsmittels benötigten Bedingungen (z.B. höhere Temperaturen) die Qualität des eigentlichen Produkts beeinträchtigen.

Die DE 4220239 A1 aus dem Jahr 1993 beschreibt eine Mischvorrichtung mit welcher aus zwei oder mehr vorzugsweise reaktionsfähigen, flüssigen Komponenten homogene und stabile Gemische erhalten werden können. Als Beispiel für eine flüssige Komponente werden Lösemittel genannt, in welchen weitere organische Produkte gelöst sein können. Die DE 4220239 A1 fokussiert dabei auf den Mischvorgang. Eine bewusste und gewollte Reaktionsführung in der Mischvorrichtung selbst, insbesondere zwischen Michael-Donoren und Michael-Akzeptoren im Sinne dieser Erfindung wird hingegen nicht beschrieben. Die Eignung dieser Mischvorrichtung als Reaktor selbst, in welchem ein signifikanter Anteil der reaktionsfähigen Komponenten zur Reaktion gebracht wird, ist ebenfalls nicht offenbart. Ebenso wenig wird auf die für eine gezielte Reaktionsführung notwendige Möglichkeit einer externen Temperierung durch Heiz- oder Kühlaggregate eingegangen.

Die WO 2010/133292 A1 hingegen offenbart ein Verfahren zur kontinuierlichen Herstellung von Epoxy-Amin-Verbindungen. Zur Durchführung des Verfahrens können gemäß der WO 2010/133292 A1 beliebige kontinuierlich betreibbare Reaktoren verwendet werden, wobei lediglich in einer speziellen Ausführungsform eine Mischpumpe eingesetzt wird, in welcher auch ein Teil der Reaktion zwischen Epoxidkomponente und Aminkomponente ablaufen kann. Eine Übertragbarkeit dieser speziellen Verfahrenstechnik auf die Michael-Addition der vorliegenden Erfindung mit den der Michael-Addition typischen Problemen einer möglichen Autopolymerisation der eingesetzten alkenisch oder alkinisch ungesättigten Verbindungen wird in WO 2010/133292 A1 weder offenbart noch in irgendeiner Weise angeregt. Für den auf dem Gebiet der Michael-Addition bewanderten Durchschnittsfachmann gab es somit keinerlei Veranlassung eine verfahrenstechnische Maßnahme in Erwägung zu ziehen, die für eine Reaktion zwischen solchen Komponenten beschrieben wurde, die per se die Problematik einer Autopolymerisation nicht kennt.

Aufgabe der vorliegenden Erfindung war es ein Verfahren zur kontinuierlichen Herstellung von Michael-Additionsprodukten bereitzustellen, welches die Nachteile des Stands der Technik nicht aufweist. Insbesondere sollten lokale Überhitzungen vermieden werden, um die geforderten möglichst hohen Produktqualitäten zu gewährleisten und durchgehende Reaktionen wie beispielsweise ein Polymerisationsrisiko zu vermeiden. Dabei sollte trotzdem eine hohe Raum-ZeitAusbeute erreicht werden.

Mischtechniken, die es erforderlich machen, in mehreren Reaktorabschnitten oder miteinander kombinierten Reaktoren mehrere aktive Mischeinrichtungen zu implementieren, sollten insbesondere aus Wartungs- und Wirtschaftlichkeitsgründen vermieden werden. Trotzdem sollte das Verfahren gewährleisten, dass nicht nur ineinander lösliche, niedermolekulare oder niederviskose Edukte einsetzbar sind. Vielmehr sollten auch dann Produkte in hoher Qualität und guter Ausbeute erhältlich sein, wenn diese aus hochviskosen Edukten erhalten werden oder Edukten, die ineinander schlecht löslich sind, ohne dass größere Mengen Lösemittel zum Einsatz kommen müssen.

Das Verfahren soll zudem eine rasche und exakte Einstellung der Reaktionsbedingungen ermöglichen, um bei verkürzter Reaktionszeit trotzdem verbesserte oder zumindest im wesentlichen gleiche Selektivitäten und Ausbeuten zu erhalten, als dies mit den Verfahren des Stands der Technik möglich ist. Es sollte durch die verkürzten Reaktionszeiten des bereitzustellenden Verfahrens auch die Möglichkeit eröffnet werden, die Reaktionen bei deutlich höheren Temperaturen durchzuführen als dies bei diskontinuierlichen Verfahren aufgrund der langen Reaktorverweilzeiten möglich ist. Auch sollte die Möglichkeit bestehen, gasförmige mit flüssigen Edukten zur Reaktion zu bringen. Insbesondere sollte das Verfahren auch eine sichere Herstellung der Zielprodukte ermöglichen, wobei diese Herstellung in Abwesenheit von Lösungsmitteln möglich sein sollte.

Die oben genannten sowie die darüber hinaus in der folgenden Beschreibung angesprochenen Aufgaben und Probleme konnten gelöst werden durch Bereitstellung eines Verfahrens zur kontinuierlichen Herstellung von Reaktionsprodukten durch Additionsreaktion, wobei mindestens eine Verbindung (B), die mindestens eine nukleophile funktionelle Gruppe aufweist, an mindestens eine Verbindung (A) addiert wird, die mindestens eine aktivierte alkenische oder aktivierte alkinische Kohlenstoff-Kohlenstoff-Mehrfachbindung aufweist, wobei die Reaktion in einer Reaktionsmischpumpe erfolgt.

Bei dieser Art von Additionsreaktion handelt es sich um eine Additionsreaktion auf Basis der Michael-Reaktion. Von Additionsreaktionen der vorliegenden Erfindung sind Hydrosilylierungsreaktionen zu unterscheiden, die schon deshalb nicht erfindungsgemäß sind, da bei einer Hydrosilylierung die funktionelle Gruppe Si-H aufgrund der Elektropositivität des Siliziumatoms nicht als Donor für eine Kohlenstoff-Kohlenstoff-Mehrfachbindung agieren kann. Bei den Additionsreaktionen auf Basis der Michael-Reaktion gemäß der vorliegenden Erfindung werden daher ganz besonders bevorzugt Kohlenstoff-Kohlenstoff-, Kohlenstoff-Stickstoff-, Kohlenstoff-Schwefel- oder Kohlenstoff-Phosphor-Einfachbindugnen neugebildet.

Reaktionsmischpumpen im Sinne dieser Erfindung sind vorzugsweise vom Peripheralradpumpen-Typ und sind ausgestattet mit
(a) einer rotationssymmetrischen Mischkammer aus einer Umfangswand und zwei Stirnseiten, die strömungstechnisch miteinander verbundene ringförmige Kanäle aufweisen,
(b) mindestens einer Einlassöffnung zur Mischkammer, worüber die Verbindung(en) (A) zugeführt werden,
(c) mindestens einer Einlassöffnung zur Mischkammer, worüber Verbindung(en) (B) zugeführt werden,
(d) einem magnetgekuppelt angetriebenen Mischrotor in der Mischkammer, der stirnseitig symmetrisch angeordnete Kantenbrüche aufweist, die mit den ringförmigen Kanälen an den Stirnseiten der Mischkammer Druckzellen bilden und wobei die Druckzellen über Durchgangsbohrungen im Mischrotor miteinander verbunden sind,
(e) einer Auslassöffnung der Mischkammer, worüber das Reaktionsgemisch und/oder das Produkt aus der Reaktionsmischpumpe ausgetragen werden, und
(f) einem durch ein externes Heiz- beziehungsweise Kühlaggregat temperierbaren Temperierkreislauf.

Eine für das erfindungsgemäße Verfahren geeignete Rotationsmischpumpe wird beispielsweise in der bereits oben genannten DE-A-42 20 239 beschrieben, welche durch Zitieren in diese Anmeldungen aufgenommen wird. Der Pumpenkopf ist jedoch zusätzlich für den Zweck der vorliegenden Erfindung über einen Temperierkreislauf durch ein externes Heiz- bzw. Kühlaggregat temperierbar ausgeführt. Die Peripherie besteht zusätzlich mindestens aus einer gegebenenfalls beheizbaren Dosiereinrichtung für jedes Edukt und einer nachgeschalteten gegebenenfalls beheizbaren Leitung für das Reaktionsgemisch.

Verschiedene weitere Ausführungen erfindungsgemäß einsetzbarer Reaktionsmischpumpen sind beispielsweise von der Firma K-ENGINEERING (Westoverledingen, Deutschland) unter der Bezeichnung "HMR" kommerziell erhältlich. Diese Vorrichtungen vereinigen in sich die Eigenschaften einer Peripheralrad-Pumpe, eines Mischers zur besonders effektiven Durchmischung sowie eines Reaktors. Hierdurch bedarf das erfindungsgemäße Verfahren eines sehr geringen apparativen Aufwandes. Die Mischkammer der erfindungsgemäß einsetzbaren Reaktionsmischpumpe(n) umfassen einen Lagerträger und ein zylindrisches Einsatzelement mit einer den Mischrotor übergreifenden Umfangswand. In der Umfangswand der Reaktionsmischpumpe befindet sich je mindestens eine Einlassöffnung für die Verbindungen (A) und Verbindungen (B), sowie eine Auslassöffnung für das Reaktionsgemisch.

Als besonders günstig hat es sich erwiesen, die Eintrittsöffnungen der Reaktionspumpen in Richtung auf die Reaktionsmischkammer düsenartig verjüngt zu gestalten, weil dadurch eine Art Saugeffekt entsteht.

Die Umdrehungsfrequenz des Rotors, welche zweckmäßigerweise über einen externen Frequenzumrichter gesteuert wird, beträgt bei der Durchführung des erfindungsgemäßen Verfahrens üblicherweise 50 bis 50000 Umdrehungen pro Minute.

Das Reaktionsvolumen in der Reaktionsmischpumpe beträgt üblicherweise 1 bis 1000 cm³, vorzugsweise 1 bis 100 cm³ und besonders bevorzugt 5 bis 100 cm³.

Selbstverständlich müssen die Teile der Reaktionsmischpumpe, die mit den Edukten, dem Reaktionsgemisch und dem Reaktionsprodukt in Berührung gelangen aus einem Material gefertigt oder damit verkleidet sein, welches sich inert gegenüber den Bestandteilen der Reaktionsmischung verhält. Solche Materialien sind beispielsweise Metalle bzw. Metallegierungen, wie Hastelloy, Titan oder Nickel, Kunststoffe, wie Polyethylen (PE), Polypropylen (PP), Polyvinylidenfluorid (PVDF) oder insbesondere Polytetrafluorethylen (PTFE), oder Oxidkeramik.

Die technische Auslegung der Reaktionspumpen erfolgt in der Regel entsprechend den gewünschten Druckverhältnissen in der Mischkammer.

Werden größere Menge des Zielprodukts gefordert, so können auch mehrere Reaktionsmischpumpen als Reaktionsmischpumpen-Aggregat parallelgeschaltet betrieben werden (im Sinne eines sogenannten "Numbering-Up"). Zu diesem Zweck wird die Reaktionsmischpumpe in einer Vorrichtung angeordnet, welche weitere jeweils unabhängig voneinander kontinuierlich betriebene Reaktionsmischpumpen enthält, in welchen die Verbindung(en) (A) mit der der oder den Verbindung(en) (B) umgesetzt werden, wobei die Reaktionsmischpumpen zeitgleich und unabhängig voneinander parallel betrieben werden können. Ein derartiger Parallelbetrieb gewährleistet nicht nur die Erzeugung von hohen Produktionsmengen, sondern auch eine hohe Flexibilität, da kurzfristig und mit verhältnismäßig geringem Aufwand eine derart betriebene defekte Reaktionsmischpumpe durch eine andere ersetzbar ist. Auch wird im Vergleich zu Batch-Verfahren eine größere Reaktorsicherheit gewährleistet, da bei technischen Problemen die Gefahr eines Austritts größerer Mengen an Edukt, Reaktionsgemisch und Produkt vermieden wird.

Die im erfindungsgemäßen Verfahren eingesetzten Reaktionsmischpumpen können darüber hinaus mit weiteren Anschlussmöglichkeiten für Heiz-, Kühl- und Spülkreisläufe ausgestattet sein.

Vorstehend beschriebene Reaktionsmischpumpen beschleunigen die Stoff- und Wärmetransportprozesse, wobei sich zusätzlich Anfangs- und Randbedingungen der Reaktion exakt einstellen lassen. Die Verweilzeiten sind besonders genau einstellbar, wobei das stark exotherme erfindungsgemäße Verfahren annähernd isotherm sowie vorzugsweise gleichzeitig bei niedriger Temperatur betrieben werden kann.

Typische Betriebsparameter der im erfindungsgemäßen Verfahren einsetzbaren Reaktionsmischpumpen sind deren Durchsatz von vorzugsweise 100 ml/h bis 1000 l/h, besonders bevorzugt 100 ml/h bis 10 l/h, die Temperatur in der Reaktionsmischpumpe von vorzugsweise -50 bis 300 °C, besonders bevorzugt 50 bis 250 °C, der Druck in der Reaktionsmischpumpe von vorzugsweise 0 bis 20 bar, besonders bevorzugt 0 bis 10 bar, die Umdrehungsgeschwindigkeit des Rotors von vorzugsweise 50 bis 50000 Umdrehungen pro Minute, besonders bevorzugt 500 bis 10000 Umdrehungen pro Minute, die Verweilzeit in der Reaktionsmischpumpe von vorzugsweise 0,1 Sekunde bis 30 min, vorzugsweise bis zu 10 min und besonders bevorzugt bis zu 1 min.

In einer besonderen Ausführungsform sind dem Reaktor weitere in kontinuierlicher Weise betriebene Reaktorsysteme nachgeschaltet, welche eine Nachdosierung der Verbindung(en) (A) und/oder der Verbindung(en) (B) und/oder eine Nachtemperierung zur Vervollständigung der Reaktion verwirklichen können. Die Nachreaktion in den nachgeschalteten Reaktorsystemen gewährleistet in einigen Fällen erst das Erreichen des gewünschten Umsatzes, welcher typischerweise bezogen auf die insgesamt umsetzbaren nukleophilen Donor-Funktionen bei vorzugsweise mindestens 30 %, besonders bevorzugt mindestens 70 % und ganz besonders bevorzugt bei 95 % und höher liegt.

Im einfachsten und bevorzugtesten Fall ist ein Nachreaktor in Form eines Rohrs oder Schlauchs ausgebildet, jeweils aus gegenüber den Edukten, dem Reaktionsgemisch und den Produkten inertem Material. Werden derart einfache Nachreaktoren verwendet, bedarf es aufgrund der bereits hervorragenden Durchmischung in der Reaktionsmischpumpe üblicherweise keiner Verwendung weiterer Mischeinrichtungen im Nachreaktor. Typische Nachreaktionszeiten liegen von 0 bis 100 min, vorzugsweise 0 bis 50 min und besonders bevorzugt 5 bis 30 min.

In einer besonderen Ausführungsform können als nachgeschaltete Reaktoren auch Reaktionsmischpumpen eingesetzt werden, wie sie im erfindungsgemäßen Verfahren Einsatz finden. Der Produktstrom aus der im erfindungsgemäßen Verfahren eingesetzten Reaktionsmischpumpe ist dann einer der Eingangsbeziehungsweise Eduktströme für den nachgeschalteten Reaktor. So können beispielsweise reaktive funktionelle Gruppen im Produkt des erfindungsgemäßen Verfahrens im Nachreaktor mit anderen Verbindungen umgesetzt werden.

Die Umsetzungsprodukte aus Donor-Verbindung(en) (B) und Akzeptor-Verbindung(en) (A) liegen in Form von Additionsverbindungen vor. Vorzugsweise werden in der Reaktionsmischpumpe 10 bis 100 Mol% der durch die Zufuhr der elektrophilen Akzeptor-Verbindung(en) (A) in den Reaktor eingeführten alkenisch oder alkinisch ungesättigten Funktionen im Reaktor umgesetzt. In manchen Ausführungsformen des Verfahrens kann es bevorzugt sein, dass beispielsweise nur 10 bis 50 Mol% oder 20 bis 50 Mol% der durch die Zufuhr der Akzeptor-Verbindung(en) (A) in den Reaktor eingeführten alkenisch oder alkinisch ungesättigten Funktionen im Reaktor umgesetzt werden. Dies gilt insbesondere dann, wenn wie oben beschrieben Nachreaktoren zum Einsatz kommen. In einigen Fällen läßt sich auf diese Weise auch die Verweilzeit in der Reaktionsmischpumpe minimieren, wenn nämlich durch die teilweise Bildung des Produkts eine Lösung oder Emulgierung der Edukte im Produkt (zusätzlich zur aktiven Vermischung durch die Pumpe) zur Homogenisierung der Reaktanten beiträgt. In allen Fällen wird jedoch ein Mindestanteil der in den Reaktor kontinuierlich eingeführten Verbindungen mit alkenisch oder alkinisch ungesättigten Gruppen, hauptsächlich (oder gegebenenfalls auch ausschließlich) mit der oder den Verbindungen(en) (B) noch in der Reaktionsmischpumpe selbst reagieren. Diesbezüglich gewährleistet das erfindungsgemäße Verfahren dem Reaktionsgemisch im Reaktor eine genügend lange Verweilzeit. Zur Bestimmung des Umsetzungsgrades eignen sich alle gängigen optischen Analyseverfahren wie beispielsweise Raman-, UV-, IR- oder NIR-Spektroskopie sowie auch NMR-Spektroskopie, insbesondere gekoppelt mit chromatographischen Verfahren wie Gelpermeationschromatographie oder HPLC. Insbesondere eignet sich die UV- sowie die NIR-spektroskopische Beobachtung des Verschwindens der Bande der Kohlenstoff-Kohlenstoff-Mehrfachbindung.

Wesentlich ist, dass das erfindungsgemäße Verfahren verhältnismäßig leicht handhabbar ist, wodurch die zugrunde liegende stark exotherme Reaktion gut kontrollierbar ist. Insbesondere im Dauerbetrieb gewährleistet das erfindungsgemäße Verfahren eine hohe Wirtschaftlichkeit und Betriebssicherheit. Gleichzeitig wird die unerwünschte Bildung von Nebenprodukten durch radikalische Polymerisation unterbunden.

Über die Verbindung(en) (B) werden eigenschaftsmodifizierende Reste in die alkenisch oder alkinisch ungesättigten Verbindungen (A) eingebracht, die mit über den Einsatzzweck der Zielprodukte entscheiden.

Als Verbindung(en) (A) werden Verbindungen eingesetzt, die aktivierte Kohlenstoff-Kohlenstoff-Mehrfachbindungen aufweisen. Geeignete Kohlenstoff-Kohlenstoff-Mehrfachbindungen sind C=C-Doppelbindungen und C=C-Dreifachbindungen, wobei solche Verbindungen bevorzugt eingesetzt werden, die eine oder mehrere C=C-Doppelbindungen aufweisen. Die Kohlenstoff-Kohlenstoff-Mehrfachbindungen können in den Verbindungen (A) terminal oder an anderer Stelle in der Verbindung vorliegen, wobei terminale Kohlenstoff-Kohlenstoff-Mehrfachbindungen bevorzugt sind. Besonders bevorzugt sind Verbindungen (A), die terminale C=C-Doppelbindungen enthalten.

Keine Kohlenstoff-Kohlenstoff-Mehrfachbindungen im Sinne dieser Erfindung sind aromatische Kohlenstoff-Kohlenstoff-Bindungen. So liegt beispielsweise im Benzylacrylat nur eine Kohlenstoff-Kohlenstoff-Mehrfachbindung im Sinne der Erfindung vor, nämlich die des Acrylat-Restes im Benzylacrylat.

Die Verbindungen (A) lassen sich vorzugsweise durch die allgemeinen Strukturformeln (Ia) und (Ib) darstellen: 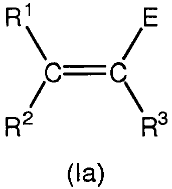 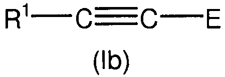 worin
E für einen elektronenziehenden Substituenten steht und R¹, R² und R³ unabhängig voneinander für H, E, einen aliphatischen, aromatischen oder aliphatisch-aromatischen Rest stehen und wobei R¹ und E durch Ringschluss miteinander verbunden sein können. Der elektronenziehende Substituent E aktiviert durch seine Anwesenheit die benachtbarte C=C-Doppelbindung beziehungsweise C=C-Dreifachbindung im Sinne der vorliegenden Erfindung.

Werden bestimmte Reste - wie beispielsweise die oben genannten Reste R¹, R² und R³ oder die weiter unten genannten Reste R, R⁴, R' und R^{z} - in der vorliegenden Erfindung allgemein als aliphatisch, aromatisch oder aliphatisch-aromatisch bezeichnet, so kann es sich generell um monomere, oligomere oder polymere Reste handeln. Soweit nicht ausdrücklich anderes erwähnt wird, können alle Reste substituiert oder unsubstituiert sein und es kann sich auch um oligomere oder polymere Reste handeln.

Bevorzugte elektronenziehende Substituenten E sind beispielsweise COR, COOR, CONHR, CONR₂, CN, PO(OR)₂, Pyridyl, SOR, SOOR, F oder NO₂, wobei die Reste R unabhängig voneinander für H oder einen aliphatischen, aromatischen oder aliphatisch-aromatischen Rest stehen. Ganz bevorzugt enthält der elektronenziehende Substituent E eine Mehrfachbindung, die in Konjugation mit der aktivierten Kohlenstoff-Kohlenstoff-Mehrfachbindung steht, so dass letztere eine aktivierte konjugierte Kohlenstoff-Kohlenstoff-Mehrfachbindung ist. Vorzugsweise steht R für einen aliphatischen Rest, besonders bevorzugt einen verzweigten oder unverzweigten Alkylrest mit 1 bis 12 Kohlenstoffatomen. In einer anderen bevorzugten Ausführungsform steht R für einen aromatischen Rest mit 6 bis 10 Kohlenstoffatomen, wie beispielsweise für einen Phenylrest. In einer weiteren bevorzugten Ausführungsform steht R für einen aliphatisch-aromatischen Rest, wie beispielsweise einen Benzylrest. In einer weiteren besonders bevorzugten Ausführungsform handelt es sich beim Rest R um einen Polyether-Rest, einen Polyester-Rest oder einen Polyether-Polyester-Rest. Die Reste R selbst können substituiert oder unsubstituiert sein. Als Substituenten kommen insbesondere funktionelle Gruppen wie beispielsweise Carbonsäuregruppen, Carbonsäureestergruppen, Carbonsäureamidgruppen und Hydroxylgruppen in Frage.

R¹, R² und R³ in den allgemeinen Formeln (la) und (Ib) stehen unabhängig voneinander für H, E, einen aliphatischen, aromatischen oder aliphatisch-aromatischen Rest, gemäß der vorstehenden Definitionen für R.

Bevorzugt sind die alkenisch ungesättigten Verbindungen der allgemeinen Formel (Ia). Unter diesen sind besonders solche bevorzugt, für welche gilt, dass mindestens zwei der Reste R¹, R² und R³ für Wasserstoff stehen.

Stehen alle drei Reste R¹, R² und R³ in der allgemeinen Formel (la) für Wasserstoff, so spricht man von vinylisch ungesättigten Verbindungen (la). Vinylisch ungesättigte Verbindungen sind erfindungsgemäß ganz besonders bevorzugt. Typische und bevorzugte Vertreter hierunter sind, Acrylsäure, Acrylsäureester (=Acrylate), Acrylsäureamide (=Acrylamide), Acrylnitril, Vinylsulfone, Vinylphosphonate, Vinylketone, Vinylaldehyde, Vinylpyridine und Nitroethylen.

Stehen die Reste R¹ und R² in der allgemeinen Formel (la) für Wasserstoff, R³ für Methyl und E für einen Rest COOR, CONHR, CONR₂, CN (jeweils wie oben definiert), so spricht man von Methacrylsäure (R=H) und deren Estern, Amiden und Nitrilen. Typische und bevorzugte Vertreter hierunter sind, Methacrylsäure, Methacrylsäureester (=Methacrylate), Methacrylsäureamide (=Methacrylamide) und Methacrylnitril. Ist ein Wasserstoffatom am Methylrest R³ substituiert, beispielsweise durch eine weitere elektronenziehende Gruppe E, so gelangt man im Fall E = COOR, CONHR oder CONR₂ zur ebenfalls einsetzbaren Itaconsäure (R=H), deren Estern oder Amiden.

Bei einer weiteren bevorzugten Ausführungsform, bei welcher ebenfalls genau zwei der drei Reste R¹, R² und R³ für Wasserstoff stehen, steht der nicht für Wasserstoff stehende dritte Rest für eine weitere Gruppe E. Ein typisches Beispiel hierfür sind die Cyanacrylate (R¹=R²=H, R³=CN und E=COOR) bzw. die Maleinsäure, deren Ester und Amide (R²=R³=H, R¹=COOR oder CONHR oder CONR₂, E= COOR oder CONHR oder CONR₂). Unter diese Ausführungsform fällt auch die bevorzugte Möglichkeit der Verbindung der Reste R¹ und E durch Ringschlussbildung. So können die Reste R¹ und E beispielsweise den gemeinsamen Rest CO-NR-CO bilden, was im Falle von (R²=R³=H) zu den ganz besonders bevorzugten Maleimiden führt (siehe untenstehende Formel (Ia')).

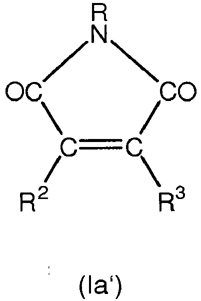

Unter den Verbindungen der allgemeinen Formel (Ib) sind solche bevorzugt, bei welchen E eine Ketogruppe oder Estergruppe ist. R¹ in Verbindungen der allgemeinen Formel (Ib) steht vorzugsweise ebenfalls für E.

Typische Vertreter der Verbindungen (Ib) sind beta-Ketoacetylene und Acetylenester (Mono- und Di-Ester).

Vorzugsweise handelt es sich bei den Verbindungen (A) um solche, die gewählt sind aus der Gruppe umfassend aus (Meth)acrylat, (Meth)acrylamid, Acrylnitril, Maleinsäure, deren Ester, Amide und Imide, Itaconsäure, deren Ester und Amide, Cyanacrylate, Vinylsulfone, Vinylphosphonate, Vinylketone, Nitroethylene, α,β-ungesättigte Aldehyde, Vinylpyridine, β-Ketoacetylene und Acetylenester. Wie üblich umfasst auch hier die Schreibweise "(Meth)acryl" sowohl "Acryl" als auch "Methacryl".

Besonders bevorzugt sind die Verbindungen (A) gewählt aus der Gruppe umfassend (Meth)acrylate, (Meth)acrylamide, Maleinsäureester und Maleimide und Vinylphosphonate.

Ganz besonders bevorzugt sind die in den Verbindungen (A) gewählt aus der Gruppe umfassend Acrylate, Acrylamide und Maleinsäureester.

Die Verbindungen (A) sind dabei bevorzugt solche, die die Kohlenstoff-Kohlenstoff-Mehrfachbindungen in endständiger (terminaler) Position aufweisen.

Besonders bevorzugt enthalten die Verbindungen (A) zwei oder mehrere Reste CR¹R²=CR³COO, wobei R¹, R² und R³ unabhängig voneinander wie oben definiert sind. Diese Reste CR¹R²=CR³COO sind dann über den Rest E in die Verbindungen (A) der allgemeinen Formel (la) eingeführt, wobei E für COOR steht und der Rest R ein oder mehrere der Gruppen CR¹R²=CR³COO enthält. Beispiele derartiger Verbindungen (A) mit zwei Resten CR¹R²=CR³COO sind Hexandioldiacrylat (HDDA), Dipropylenglykoldiacrylat (DPGDA) und Tripropylenglykoldiacrylat (TPGDA). Solche mit drei oder vier Resten CR²=CR³COO sind beispielsweise Trimethylolpropantriacrylat (TMPTA), Pentaerythrittriacrylat (PETA), Pentaerythrittetraacrylat und Ditrimethylolpropantetraacrylat (DTMPTTA). In den vorgenannen Fällen handelt es sich bei den Resten R, wenn E für COOR steht, um kurzkettige monomere aliphatische Reste vorzugsweise mit einem zahlenmittleren Molekulargewicht kleiner 1500 g/mol, besonders bevorzugt kleiner 1000 g/mol, ganz besonders bevorzugt kleiner 500 g/mol, die ein, zwei oder drei CR¹R²=CR³COO-Gruppen tragen. Die vorgenannten Verbindungen (A) werden häufig auch als sogenannte Reaktiwerdünner bezeichnet (siehe beispielsweise Römpp Lexikon Lacke & Druckfarben, Georg Thieme Verlag 1998, Seite 491, Stichwort "Reaktivverdünner").

Es ist jedoch auch möglich, dass es sich beim Rest R, wenn E für COOR steht, um eine oligomere oder polymere Gruppe handelt, beispielsweise um einen Polyether- oder Polyester-Rest, der weitere CR¹R²=CR³COO-Gruppen trägt. In einem solchen Fall handelt es sich vorzugsweise um Polyetheracrylate oder Polyesteracrylate. Beispiele für Polyetheracrylate sind Polyethylenglykolmonoacrylate und Polyethylenglykoldiacrylate, Polypropylenglykolmonoacrylate und Polypropylenglykoldiacrylate, gemischte Polyethylenglycol/propylenglycolmono- und diacrylate, Neopentylglykoldiacrylat, Diacrylate von alkoxyliertem Neopentylglykol, Glycerintriacrylat, Triacrylate von alkoxyliertem Glycerin, Trimethylolpropantriacrylat, Triacrylate von alkoxyliertem Trimethylolpropan, Bisphenol A-Diacrylat oder Diacrylate von alkoxyliertem Bisphenol A. Einsetzt werden können jedoch auch sogenannte Epoxyacrylate oder Urethanacrylate. Weiterhin ist es möglich, dass es sich beim Rest R, wenn E für COOR steht, um einen Rest enhaltend eine Polysiloxankette, bevorzugt eine Polydimethylsiloxankette handelt.

Monomere, oligmere oder polymere Verbindungen (A) enthalten in einer besonders bevorzugten Ausführungsform mindestens zwei (Meth)acrylatgruppen, noch bevorzugter 2 bis 8 oder ganz besonders bevorzugt 2 bis 4 (Meth)acrylatgruppen.

Verbindungen (B) sind nukleophile Donor-Verbindungen.

Die Verbindungen (B) lassen sich vorzugsweise durch die allgemeine Strukturformel (II) darstellen:

R⁴-D-H (II)

worin R⁴ für einen aliphatischen, aromatischen oder aliphatisch-aromatischen Rest steht und D für CR'E, NR', PR' oder S steht, wobei R' für Wasserstoff oder einen aliphatischen, aromatischen oder aliphatisch-aromatischen Rest und E für einen elektronenziehenden Substituenten steht. Der elektronenziehende Substituent ist wie in den allgemeinen Strukturformeln (la) und (Ib) definiert. Der elektronenziehende Substituent E im Rest CR'EH ermöglicht die Deprotonierung des am Kohlenstoffatom gebundenen Wasserstoff-Rests, der in der chemischen Fachliteratur auch als CHacider Wasserstoff bezeichnet wird, unter Bildung eines sogenannten Carbanions. Wie Eingangs bereits erwähnt sind Carbanionen Michael-Donoren im engeren Sinne. Handelt es sich beim elektronenziehenden Substituenten E um COR, COOR, CONHR oder CONR₂, wobei die Reste R unabhängig voneinander für H oder einen aliphatischen, aromatischen oder aliphatisch-aromatischen Rest stehen, so kann sich die Carbanion-Struktur unter Einbeziehung der Gruppierung CO in die tautomere Enolat-Struktur umlagern. Besonders bevorzugt steht D für NR', PR' oder S, ganz besonders bevorzugt für NR' oder S. Der Rest R⁴ kann selbst wiederum D-H Gruppen enthalten. Steht in der allgemeinen Formel (II) D-H für eine Aminogruppe und enthält der Rest R⁴ weitere D-H Gruppen in Form von Aminogruppen, so handelt es sich bei den Verbindungen der allgemeinen Formel (II) um Polyamine. Typische Polyamine, die unter die Formel (II) fallen sind beispielsweise Diethylentriamin und Triethylentetramin. Es ist auch möglich, dass es sich um Polyamine mit unterschiedlich reaktiven Aminogruppen handelt, beispielsweise um gemischtprimär/sekundäre Amine wie N-Methylaminopropylamin (bei denen die primäre Aminogruppe der Addition im allgemeinen schneller zugänglich ist) oder um gemischt-primär/tertiäre Amine wie N,N-Dimethylaminopropylamin, bei denen eine Aminogruppe für die Addition zur Verfügung steht und die andere (tertiäre) Aminogruppe nicht mehr an eine aktivierte C-C-Mehrfachbindung addieren kann.

Es ist weiterhin möglich und in vielen Fällen bevorzugt, dass der Rest R⁴ andere funktionelle Gruppen wie beispielsweise Silylgruppen, insbesondere hydrolysierbare Silylgruppen enthält. Ein bevorzugter Rest R⁴ ist beispielsweise ein Rest R⁵-Si(R⁶)ₙ (R⁷)₃₋ₙ worin R⁵ ein Alkylen-Rest mit 1 bis 6, vorzugsweise 1 bis 3 Kohlenstoffatomen ist, R⁶ ein durch Hydrolyse abspaltbarer Rest, beispielsweise ein Halogenrest, ein Alkoxyrest mit 1 bis 4 Kohlenstoffatomen oder ein Acetyl-Rest ist, R⁷ ein Alkyl-Rest mit 1 bis 6 Kohlenstoffatomen ist und n = 1 bis 3 ist. Insbesondere sind hydrolysierbare Silylgruppen im Rest R⁴ dann bevorzugt, wenn es sich beim Rest D um eine Gruppe NR' oder um S handelt.

Eine weitere funktionelle Gruppe, die der Rest R⁴ aufweisen kann, sind Hydroxygruppen. Dies ist insbesondere dann bevorzugt, wenn es sich beim Rest D um eine Gruppe NR' handelt, so dass es sich bei der Michael-Additionsreaktion um die Addition eines sogenannten Aminoalkohols an eine aktivierte Kohlenstoff-Kohlenstoff-Mehrfachbindung handelt.

Insbesondere zählen zu den nukleophilen Donor-Verbindungen (B) primäre und sekundäre Amine, Thiole, Phosphine, und Carbanionen-bildenden Verbindungen. Besonders bevorzugte Verbindungen (B) sind primäre und sekundäre Amine.

Beim Einsatz verschiedener Verbindungen (A) und/oder (B) können die verschiedenen Verbindungen (A) vorvermischt einer Einlassöffnung der Reaktionsmischpumpe zugeführt werden oder über getrennte Einlassöffnungen. Gleiches gilt für die Verbindungen (B).

Die Umsetzung der Verbindung(en) (A) mit der oder den Verbindung(en) (B), kann in einem Lösungsmittelsystem, bevorzugt aber in Substanz nach den dem Fachmann bekannten Verfahren durchgeführt werden. Unter einer Reaktion "in Substanz" wird hierin eine solche verstanden, die ohne oder weitestgehend ohne Lösemittelzusatz erfolgt, wobei geringe Mengen Lösemittel (kleiner 5 Gew.-%, insbesondere kleiner 2 Gew.-% bezogen auf das Gewicht der Reaktionsmischung) - beispielsweise über einen ggf. verwendeten Katalysator oder zur Erhöhung der Reaktionsgeschwindigkeit infolge Polaritätserhöhung in die Reaktionsmischung eingebracht - unberücksichtigt bleiben. Werden Lösemittel eingesetzt, so dienen diese insbesondere zur Viskositätsanpassung oder zur Erhöhung der Reaktionsgeschwindigkeit. Die zu wählende Reaktionstemperatur hängt dabei auch von der Reaktivität der Edukte ab. Gegebenenfalls werden dem Fachmann bekannte Katalysatoren verwendet, um die Umsetzung zu beschleunigen.

Typische Katalysatoren der erfindungsgemäßen Additionsreaktionen sind basische oder saure Katalysatoren, Phosphine oder Lantanoid-Verbindungen. Unter den basischen Katalysatoren besonders bevorzugt sind tertiäre Amine. Als saure Katalysatoren können vor allem Lewis-Säuren wie Bortrifluorid, Zinkchlorid, Aluminiumchlorid oder Titantetrachlorid zum Einsatz kommen. Lanthanoid-Verbindungen sind z.B. in DE 69607568 beschrieben.

Vorteilhaft werden die Katalysatoren in der oder den Verbindung(en) (A) oder (B) vor Eintritt in die Reaktionsmischpumpe gelöst oder dispergiert.

Im bevorzugten Fall, in dem Amine als Nukleophil eingesetzt werden, kann vorzugsweise der Zusatz eines Katalysators auch entfallen, da die Aminoverbindungen bzw. ihre Umsetzungsprodukte selbst katalytisch wirken.

In einer Ausführungsform werden Verbindung(en) (A) und Verbindung(en) (B) in einem solchen Verhältnis der Reaktionsmischpumpe zugeführt, dass das Verhältnis von Kohlenstoff-Kohlenstoff-Mehrfachbindungen aus Verbindung(en) (A) zu nukleophilen Gruppen aus Verbindung(en) (B) im Wesentlichen äquimolar ist, d. h. 1:1,1 bis 1,1:1 beträgt.

In einer anderen Ausführungsform kann es vorteilhaft sein, Verbindung(en) (A) und Verbindung(en) (B) in einem solchen Verhältnis der Reaktionsmischpumpe zuzuführen, dass das Verhältnis von Kohlenstoff-Kohlenstoff-Mehrfachbindungen aus Verbindung(en) (A) zu nukleophilen Funktionalitäten aus Verbindung(en) (B) so gewählt ist, dass die alkenisch oder alkinisch ungesättigten Funktionalitäten in einem Überschuß, insbesondere einem Überschuß von mehr als 10 Mol-% vorliegen. Das Endprodukt enthält dann noch alkenisch oder alkinisch ungesättigte Funktionen, die
(a) für die endgültige Verwendung des Produkts, beispielsweise radikalisch polymerisierenden bzw. vernetzenden Systemen, benötigt werden oder
(b) sich mit der gebildeten Spezies weiter umsetzen oder
(c) in einer nachgeschalteten Reaktionsmischpumpe mit einer oder mehreren weiteren Verbindung(en) (A) umgesetzt werden, die sich von der oder den im ersten Schritt eingesetzten Verbindung(en) (A) unterscheiden. Auf diese Weise lassen sich auch mehr als zwei Reaktoren bzw. Reaktionsmischpumpen in Reihe betreiben.

Ein bevorzugtes Beispiel für den Fall (b) stellte die Addition von primären Aminogruppen an aktivierte Kohlenstoff-Kohlenstoff-Doppelbindungen dar, die nach der Addition als sekundäre Aminogruppen vorliegen. Diese sekundären Aminogruppen sind ebenfalls zu einer Additionsreaktion an eine aktivierte Kohlenstoff-Kohlenstoff-Doppelbindung befähigt, so dass hier Mehrfach-Additionsprodukte herstellbar sind.

In einer anderen Ausführungsform kann es vorteilhaft sein, Verbindung(en) (A) und Verbindung(en) (B) in einem solchen Verhältnis der Reaktionsmischpumpe zuzuführen, dass das Verhältnis von Kohlenstoff-Kohlenstoff-Mehrfachbindungen aus Verbindung(en) (A) zu nukleophilen Funktionalität aus Verbindung(en) (B) so gewählt ist, dass nukleophilen Funktionalitäten in einem Überschuss, insbesondere einem Überschuss von mehr als 10 Mol-% vorliegen; dies kann in dem Falle vorteilhaft sein, wenn polyfunktionelle Verbindungen (A), z.B. Polyamine, vorliegen, von denen nur ein Teil der funktionellen Gruppen umgesetzt wird. Das Endprodukt enthält dann noch nukleophile Donor-Gruppen, die
(a) für die endgültige Verwendung des Produkts, beispielsweise in Epoxidhaltigen oder Isocyanat-haltigen Bindemittelsystemen oder in radikalisch polymerisierenden bzw. vernetzenden (bevorzugt acrylatfunktionellen) Systemen, benötigt werden oder
(b) in einer nachgeschalteten Reaktionsmischpumpe mit einer oder mehreren weiteren Verbindung(en) (B) umgesetzt werden, die sich von der oder den im ersten Schritt eingesetzten Verbindung(en) (B) unterscheiden. Auf diese Weise lassen sich auch mehr als zwei Reaktoren bzw. Reaktionsmischpumpen in Reihe betreiben.

In einer bevorzugten Ausführungsform der Erfindung beträgt die Temperatur des Reaktionsgemischs im Reaktor 0-200 °C, bevorzugt 10-150 °C sowie besonders bevorzugt 20-120 °C.

Der Quotient aus Gesamtvolumen des in dem Reaktor enthaltenen Reaktionsgemischs und dem Gesamtvolumenstrom des in Form des Produktstroms aus dem Reaktor ausgeleiteten Reaktionsgemischs ist als ein Maß für die Verweilzeit anzusehen. Die relevanten verhältnismäßig kurzen Verweilzeiten gewährleisten, dass trotz der relativ hohen Temperaturen sich in einem nur geringen Maße unerwünschte Nebenreaktionen bemerkbar machen.

Die Verbindung(en) (A) sowie die Verbindung(en) (B) werden jeweils normalerweise mit einer Eintrittstemperatur von 0-200 °C, bevorzugt 10-100 °C sowie besonders bevorzugt 20-50 °C dem Reaktor zugeführt. Die Differenz zwischen der Austrittstemperatur (beim Austritt aus dem Reaktor) des Reaktionsgemischs und dieser Eintrittstemperatur beträgt meist 0 bis 100° C, bevorzugt 10 bis 50 °C. Typischerweise beträgt die Heizleistung bezogen auf die im Reaktor dem Reaktionsgemisch von außen zugeführte Wärme 1 bis 10000 Watt pro kg, bevorzugt 80 bis 2000, besonders bevorzugt ungefähr 100 bis 500 Watt pro kg.

Weiterer Gegenstand der Erfindung ist ein Verfahren zur Herstellung beschichteter pulver- oder faserförmiger Feststoffe, wobei in einem ersten Schritt das erfindungsgemäße Verfahren zur kontinuierlichen Herstellung von Reaktionsprodukten durch Additionsreaktion durchgeführt wird und in einem anschließenden Schritt pulver- oder faserförmige Feststoffe mit dem Reaktionsprodukt des im ersten Schritt durchgeführten Verfahrens beschichtet werden. Typische pulverförmige oder faserförmige Feststoffe sind Pigmente und Füllstoffe, bevorzugt anorganische Füllstoffe, sowie auch Glas- und Kohlefasern. Nanoskalige Füllstoffe (z.B. SiO₂, Al₂O₃, ZnO, Carbon-Nanotubes) stellen ein spezielles Beispiel für anorganische Füllstoffe dar. Effektpigmente (z.B. auf Basis von Metallpigmenten, beispielsweise aus Aluminium, Zink oder Messing sowie auch Perlglanzpigmente) stellen ein weiteres spezielles Beispiel dar. Die Pigmente oder Füllstoffe wiederum können vor der Beschichtung in trockener Form vorliegen oder beispielsweise in Form von Pigmentpasten, Kosmetikzubereitungen, Tinten, Druckfarben oder Lacken.

Noch ein weiterer Gegenstand der Erfindung ist ein Verfahren zur Herstellung von Beschichtungsmitteln oder Kunststoffen, wobei in einem ersten Schritt das erfindungsgemäße Verfahren zur kontinuierlichen Herstellung von Reaktionsprodukten durch Additionsreaktion durchgeführt wird und in einem anschließenden Schritt das Reaktionsprodukt des im ersten Schritt durchgeführten Verfahrens in Beschichtungsmittelzusammensetzungen oder Kunststoffe eingearbeitet wird. Beschichtungsmittel im Sinne dieser Erfindung sind beispielsweise Lacke, Klebstoffe, Dichtstoffe, Vergussmassen, aber auch im weitesten Sinne Tinten und Druckfarben. Kunststoffe können beispielsweise thermoplastische oder duroplastische Kunststoffe sein.

Noch ein weiterer Gegenstand der Erfindung ist ein Verfahren zur Herstellung von beschichteten Werkstoffoberflächen, wobei in einem ersten Schritt das erfindungsgemäße Verfahren zur kontinuierlichen Herstellung von Reaktionsprodukten durch Additionsreaktion durchgeführt wird und in einem anschließenden Schritt das Reaktionsprodukt des im ersten Schritt durchgeführten Verfahrens zur Beschichtung bzw. Behandlung von Werkstoffoberflächen eingesetzt wird. Werkstoffoberflächen im Sinne dieser Erfindung sind beispielsweise metallische Oberflächen, Glasoberflächen, Kunststoffoberflächen oder keramische Oberflächen.

Die vorliegende Erfindung betrifft auch die Additionsverbindungen, welche mit dem vorstehend beschriebenen Verfahren hergestellt worden sind. Zu diesen Additionsverbindungen zählen insbesondere auch polymere Additionsverbindungen, wie sie beispielsweise in Prog. Polym. Sci. 2006, 31, 487 beschrieben sind.

Die vorliegende Erfindung offenbart auch die Verwendung der nach dem, vorstehend beschriebenen erfindungsgemäßen Verfahren hergestellten Additionsprodukte als Additive, beispielsweise als Haftvermittler und Kopplungsmittel, als Netz- und Dispergiermittel, als oberflächenaktive, wasserabweisende oder schmutzabweisende Mittel wie beispielsweise Antigraffiti-Mittel, Trennmittel oder Benetzungsmittel. Sie können beispielsweise aber auch zur Modifizierung von Polymeren beziehungsweise Harzen oder zur Behandlung von Pigmenten oder Füllstoffen verwendet werden.

Gerade beim Einsatz als Verlauf- und Filmbildehilfsmittel in Automobilserien- oder Automobilreparaturlacken spielen die erfindungsgemäß hergestellten Additionsverbindungen eine bedeutende Rolle. In derart qualitativ hochwertigen Systemen sollten möglichst reine Verbindungen eingesetzt werden, die auch ein Minimum an Eigenverfärbung zeigen. Dies gilt insbesondere für nicht-pigmentierte Klarlacke. Da die erfindungsgemäß erhaltenen Verbindungen unter Einsatz einer minimalen Menge an Katalysator auskommen kann diese Voraussetzung erfüllt werden.

Neben dem Einsatz als Additiv in wässrigen und/oder lösemittelhaltigen Dispersionen, insbesondere Beschichtungsmitteln wie Lacken, ist es ebenfalls möglich, pulver- oder faserförmige Feststoffe, wie Pigmente oder Füllstoffe mit den nach dem erfindungsgemäßen Verfahren erhaltenen Produkten zu beschichten.

Somit offenbart die vorliegende Erfindung schließlich auch pulver- oder faserförmige Feststoffe, die mit den nach dem erfindungsgemäßen Verfahren erhaltenen Produkten beschichtet sind.

Derartige Beschichtungen von organischen und anorganischen Feststoffen werden in bekannter Art und Weise durchgeführt. Beispielsweise sind in der EP-A 0 270 126 solche Verfahren beschrieben. Speziell bei Pigmenten kann eine Beschichtung der Pigmentoberfläche während oder nach der Synthese der Pigmente erfolgen, beispielsweise durch Zusatz der erfindungsgemäß erhaltenen Produkte zur Pigmentsuspension. Auf diese Weise vorbehandelte Pigmente zeigen eine leichte Einarbeitbarkeit in das Bindemittelsystem, ein verbessertes Viskositäts- und Flockulationsverhalten sowie einen guten Glanz gegenüber nicht behandelten Pigmenten.

Generell eignen sich die erfindungsgemäßen Additionsprodukte als grenzflächenaktive Verbindungen in Lacken, Kunststoffen, Klebstoffen, Pigmentpasten, Dichtstoffen, Kosmetikzubereitungen, Keramiken, Vergussmassen, Druckfarben oder Tinten. Der Einsatz als grenzflächenaktive Verbindung kann gemäß der betreffenden Anwendung beispielsweise als Netz- und Dispergiermittel, Haftvermitter, Kupplungsmittel (Coupling Agents), Emulgator, Trennmittel, Entschäumer, Entlüfter oder als Verarbeitungshilfsmittel erfolgen.

Im Folgenden soll die Erfindung anhand von Ausführungsbeispielen näher beschrieben werden.

### BEISPIELE

### Beispiel 1 (erfindungsgemäß):

### Reaktion eines Polyethylenglykol-200-Diacrylats (PEG200-DA) mit Aminopropyltriethoxysilan (AMEO)

Zunächst wurde eine Reaktionsmischpumpe des Typs HMR-40 (K-Engineering, Westoverledingen, Deutschland) und ein Thermostat des Typs Huber K25-CC-NR für die Nachreaktion auf eine Arbeitstemperatur von 80 °C gebracht.

Nachdem die Arbeitstemperaturen erreicht waren, wurden die Massenströme aus den Vorratsgefäßen 1 und 2 (1-AMEO): 5,9 g/min; PEG200-DA: 14,5 g/min) mittels Pumpen 1 und 2 in die Reaktionskammer der Reaktionsmischpumpe kontinuierlich gefördert. Der eingesetzte Stabilisator (2,6-Di-tert-butyl-p-kresol) wurde vorher im PEG200-DA gelöst.

Die Reaktionsmischpumpe wurde über einen Frequenzumrichter mit 30 % der maximal möglichen Umdrehungszahl betrieben. Während der Reaktion (kontinuierlich über 5 Stunden) wurde im Reaktionsraum der Reaktionsmischpumpe eine Temperatur von 78-83 °C gemessen. Zur Nachreaktion wurde das Reaktionsgemisch über einen geeigneten Schlauch aus Polytetrafluorethylen durch ein beheiztes Bad des Thermostaten geleitet.

Der verwendete Schlauch zur Nachreaktion besaß einen Innendurchmesser von 6 mm und eine Länge von 10 m. Das Gesamtsystemvolumen (Reaktionsmischpumpe und nachgeschalteter Schlauch) betrug ca. 288 ml. Die Gesamtreaktionszeit (Verweilzeit in Pumpe und Schlauch) betrug ca. 15 min.

Der mittels NMR bestimmte Umsatz von Amin betrug 100%.

### Beispiel 2 (erfindungsgemäß):

### Reaktion von Dipropylenglykoldiacrylat (DPGDA) und Trimethylolpropantriacrylat (TMPTA) mit Aminopropyltriethoxysilan (AMEO)

Zunächst wurde eine Reaktionsmischpumpe des Typs HMR-40 (K-Engineering, Westoverledingen, Deutschland) und ein Thermostat des Typs Huber K25-CC-NR für die Nachreaktion auf eine Arbeitstemperatur von 40 °C gebracht.

Nachdem die Arbeitstemperaturen erreicht waren, wurden die Massenströme aus den Vorratsgefäßen 1 und 2 (DPGDA + TMPTA im Gewichtsverhältnis 9-zu-1: 11,0 g/min; AMEO: 6,1 g/min) mittels Pumpen 1 und 2 in die Reaktionskammer der Reaktionsmischpumpe kontinuierlich gefördert. Der eingesetzte Stabilisator 2,6-Di-tert-butyl-p-kresol wurde vorher im Acrylatgemisch (DPGDA+ TMPTA) gelöst.

Die Reaktionsmischpumpe wurde über einen Frequenzumrichter mit 30% der maximal möglichen Umdrehungszahl betrieben. Während der Reaktion (kontinuierlich über 5 Stunden) wurde im Reaktionsraum der Reaktionsmischpumpe eine Temperatur von 38-43 °C gemessen. Zur Nachreaktion wurde das Reaktionsgemisch über einen geeigneten Schlauch aus Polytetrafluorethylen durch ein beheiztes Bad des Thermostaten geleitet.

Der verwendete Schlauch zur Nachreaktion besaß einen Innendurchmesser von 6 mm und eine Länge von 10 m. Das Gesamtsystemvolumen betrug ca. 288 ml. Die Gesamtreaktionszeit betrug ca. 10 min.

Der mittels NMR bestimmte Umsatz von Amin betrug 100%.

### Beispiel 3 (erfindungsgemäß):

### Umsetzung eines propoxylierten Neopentylglykol-Diacrylats (2 mol Propylenoxid pro Neopentylglykol) mit Triethylentetramin (TETA) und 2-Ethylhexylacrylat (EHA), Gewichtsverhältnis 4 : 5 : 2

Zunächst wurde eine Reaktionsmischpumpe des Typs HMR-40 (K-Engineering, Westoverledingen, Deutschland) und ein Thermostat des Typs Huber K25-CC-NR für die Nachreaktion auf Raumtemperatur 25°C gebracht.

Nachdem die Arbeitstemperaturen erreicht waren, wurden die Massenströme aus den Vorratsgefäßen 1 und 2 (TETA: 1,5g/min; Diacrylat und EHA: 3,4 g/min) mittels Pumpen 1 und 2 in die Reaktionskammer der Reaktionsmischpumpe kontinuierlich gefördert. Die Reaktionsmischpumpe wurde über einen Frequenzumrichter mit 20-40% der maximal möglichen Umdrehungszahl betrieben. Während der Reaktion (kontinuierlich über 5 Stunden) wurde im Reaktionsraum der Reaktionsmischpumpe eine Temperatur von 25-30 °C gemessen. Es erfolgt keine Nachreaktion.

Man erhält ein Produkt mit folgenden analytischen Daten: Viskosität (Platte/Kegel) 36 Pas (20°C), 2000 mPas (60°C); Dichte 1,049 g/ml; Brechungsindex 1,4851

## Patentansprüche

1. Verfahren zur kontinuierlichen Herstellung von Reaktionsprodukten durch Additionsreaktion, wobei mindestens eine Verbindung (B), die mindestens eine nukleophile Gruppe aufweist, an mindestens eine Verbindung (A) addiert wird, die mindestens eine aktivierte alkenische oder aktivierte alkinische Kohlenstoff-Kohlenstoff-Mehrfachbindung aufweist, wobei die Aktivierung der Kohlenstoff-Kohlenstoff-Mehrfachbindung durch einen elektronenziehenden Substituenten erfolgt, der benachbart zur Kohlenstoff-Kohlenstoff-Doppelbindung oder Kohlenstoff-Kohlenstoff-Dreifachbindung ist, **dadurch gekennzeichnet, dass** die Reaktion in einer Reaktionsmischpumpe erfolgt.

2. Verfahren nach Anspruch 1, **dadurch gekennzeichnet, dass** die Reaktionsmischpumpe vom Peripheralradpumpen-Typ ist und ausgestattet ist mit
(a) einer rotationssymmetrischen Mischkammer aus einer Umfangswand und zwei Stirnseiten, die strömungstechnisch miteinander verbundene ringförmige Kanäle aufweisen,
(b) mindestens einer Einlassöffnung zur Mischkammer, worüber die Verbindung(en) (A) zugeführt werden,
(c) mindestens einer Einlassöffnung zur Mischkammer, worüber Verbindung(en) (B) zugeführt werden,
(d) einem magnetgekuppelt angetriebenen Mischrotor in der Mischkammer, der stirnseitig symmetrisch angeordnete Kantenbrüche aufweist, die mit den ringförmigen Kanälen an den Stirnseiten der Mischkammer Druckzellen bilden und wobei die Druckzellen über Durchgangsbohrungen im Mischrotor miteinander verbunden sind, und
(e) einer Auslassöffnung der Mischkammer, worüber das Reaktionsgemisch und/oder das Produkt aus der Reaktionsmischpumpe ausgetragen werden
(f) einem durch ein externes Heiz- beziehungsweise Kühlaggregat temperierbaren Temperierkreislauf.

3. Verfahren nach Anspruch 2, wobei die Reaktionsmischpumpe des weiteren (g) mit einer Einlassöffnung für Spülflüssigkeiten versehen ist.

4. Verfahren nach einem der Ansprüche 1 bis 3, **dadurch gekennzeichnet, dass** die Verbindung (A), eine der allgemeinen Formeln (la) und (Ib) besitzt: 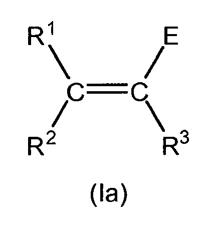 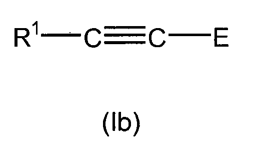 worin
E für einen elektronenziehenden Substituenten steht und R¹, R² und R³ unabhängig voneinander für H, E, einen aliphatischen, aromatischen oder aliphatisch-aromatischen Rest stehen und wobei R¹ und E durch Ringschluss miteinander verbunden sein können.

5. Verfahren nach Anspruch 4, wobei E gewählt ist aus der Gruppe bestehend aus den Resten COR, COOR, CONHR, CONR₂, CN, PO(OR)₂, Pyridyl, SOR, SOOR, F oder NO₂, wobei die Reste R unabhängig voneinander für H oder einen aliphatischen, aromatischen oder aliphatisch-aromatischen Rest stehen.

6. Verfahren nach Anspruch 4 oder 5, wobei mindestens zwei der Reste R¹, R² und R³ der Verbindung der allgemeinen Formel (la) für Wasserstoff stehen.

7. Verfahren nach einem der Ansprüche 4 bis 6, wobei
(i) die Reste R¹, R² und R³ in der allgemeinen Formel (la) für Wasserstoff stehen, oder
(ii) die Reste R¹ und R² in der allgemeinen Formel (la) für Wasserstoff stehen, R³ für einen substituierten oder unsubstituierten Methyl-Rest steht und E für einen Rest COOR, CONHR, CONR₂ oder CN steht, oder
(iii) zwei der drei Reste R¹, R² und R³ in der allgemeinen Formel (la) für Wasserstoff stehen, und der nicht für Wasserstoff stehende dritte Rest für eine weitere Gruppe E steht und die Reste R¹ und E gegebenenfalls durch Ringschlussbildung verbunden sind.

8. Verfahren nach einem der Ansprüche 1 bis 7, wobei die Verbindung (A) gewählt ist aus der Gruppe bestehend aus (Meth)acrylaten, (Meth)acrylamiden, Acrylnitrilen, Maleinsäureanhydrid, Maleinsäure, deren Estern, Amiden und Imiden, Itaconsäure, deren Estern und Amiden, Cyanacrylaten, Vinylsulfonen, Vinylphosphonaten, Vinylketonen, Nitroethylenen, α,β-ungesättigten Aldehyden, Vinylpyridinen, β-Ketoacetylenen und Acetylenestern.

9. Verfahren nach einem der Ansprüche 4 bis 8, wobei die Verbindungen (A) zwei oder mehrere Reste CR¹R²=CR³COO tragen.

10. Verfahren nach einem der Ansprüche 1 bis 9, wobei die Verbindungen (A) gewählt sind aus der Gruppe bestehend aus Hexandioldiacrylat, Dipropylenglykoldiacrylat, Tripropylenglykoldiacrylat, Trimethylolpropantriacrylat, Pentaerythrittriacrylat, Pentaerythrittetraacrylat, Dimethylolpropantetraacrylat, Polyetheracrylaten, Polyesteracrylaten, Epoxyacrylaten oder Urethanacrylaten.

11. Verfahren nach einem der Ansprüche 1 bis 10, wobei die Verbindung (B) die allgemeine Formel (II) besitzt:
R⁴-D-H (II)
worin R⁴ für einen aliphatischen, aromatischen oder aliphatisch-aromatischen Rest steht und D für CR'E, NR', PR' oder S steht, wobei R' für Wasserstoff oder einen aliphatischen, aromatischen oder aliphatisch-aromatischen Rest und E für einen elektronenziehenden Substituenten steht.

12. Verfahren nach einem der Ansprüche 1 bis 11, wobei die Verbindung (B) gewählt ist aus der Gruppe bestehend aus primären Aminen, sekundären Aminen, Thiolen, Phosphinen und Carbanionen-bildenden Verbindungen.

13. Verfahren nach Anspruch 12, wobei es sich bei den primären Aminen und sekundären Aminen um Polyamine handelt oder um weitere reaktive Reste tragende Amine, wobei die reaktiven Reste gewählt sind aus der Gruppe bestehend aus hydrolysierbaren Silylgruppen oder Hydroxylgruppen.

14. Verfahren nach einem der Ansprüche 1 bis 13, wobei die Additionsreaktion mit basischen oder sauren Katalysatoren, Phosphinen oder Lantanoid-Verbindungen katalysiert wird.

15. Verfahren zur Herstellung beschichteter pulver- oder faserförmiger Feststoffe, wobei in einem ersten Schritt das Verfahren nach einem der Ansprüche 1 bis 14 durchgeführt wird und in einem anschließenden Schritt pulver- oder faserförmige Feststoffe mit dem Reaktionsprodukt des im ersten Schritt durchgeführten Verfahrens beschichtet werden.

16. Verfahren zur Herstellung von Beschichtungsmitteln oder Kunststoffen, wobei in einem ersten Schritt das Verfahren nach einem der Ansprüche 1 bis 14 durchgeführt wird und in einem anschließenden Schritt das Reaktionsprodukt des im ersten Schritt durchgeführten Verfahrens in Beschichtungsmittelzusammensetzungen oder Kunststoffe eingearbeitet wird.

17. Verfahren zur Herstellung beschichteter Werkstoffoberflächen, wobei in einem ersten Schritt das Verfahren nach einem der Ansprüche 1 bis 14 durchgeführt wird und in einem anschließenden Schritt die Werkstoffoberfläche mit dem Reaktionsprodukt des im ersten Schritt durchgeführten Verfahrens beschichtet wird.

## Claims

1. A method for the continuous production of reaction products by addition reaction, where at least one compound (B), which has at least one nucleophilic group, is added onto at least one compound (A), which has at least one activated alkenic or activated alkynic carbon-carbon multiple bond, where the activation of the carbon-carbon multiple bond takes place by means of an electron-withdrawing substituent which is adjacent to the carbon-carbon double bond or carbon-carbon triple bond, **characterized in that** the reaction takes place in a reaction mixing pump.

2. The method as claimed in claim 1, **characterized in that** the reaction mixing pump is of the peripheral wheel pump type and is equipped with
(a) a rotationally symmetrical mixing chamber composed of a circumferential wall and two faces, which have annular channels fluidically joined to one another,
(b) at least one inlet opening to the mixing chamber, via which the compound(s) (A) are introduced,
(c) at least one inlet opening to the mixing chamber, via which compound(s) (B) are introduced,
(d) a magnet-coupling-driven mixing rotor in the mixing chamber, which has edge breaks symmetrically arranged on the face which form pressure cells with the annular channels on the faces of the mixing chamber, and where the pressure cells are joined together via connecting bores in the mixing rotor, and
(e) an outlet opening of the mixing chamber, via which the reaction mixture and/or the product are discharged from the reaction mixing pump
(f) a thermally regulatable circuit thermally regulatable by means of an external heating or cooling unit.

3. The method as claimed in claim 2, where the reaction mixing pump is provided further (g) with an inlet opening for rinse liquids.

4. The method as claimed in any one of claims 1 to 3, **characterized in that** the compound (A) has one of the general formulae (Ia) and (Ib): 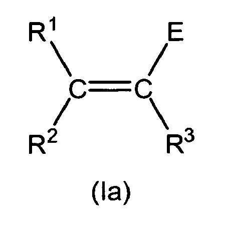 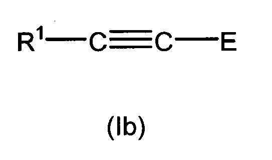 in which
E is an electron-withdrawing substituent, and R¹, R² and R³, independently of one another, are H, E, an aliphatic, aromatic or aliphatic-aromatic radical, and where R¹ and E can be joined together by ring closure.

5. The method as claimed in claim 4, where E is selected from the group consisting of the radicals COR, COOR, CONHR, CONR₂, CN, PO(OR)₂, pyridyl, SOR, SOOR, F or NO₂, where the radicals R, independently of one another, are H or an aliphatic, aromatic or aliphatic-aromatic radical.

6. The method as claimed in claim 4 or 5, where at least two of the radicals R¹, R² and R³ in the compound of the general formula (Ia) are hydrogen.

7. The method as claimed in any one of claims 4 to 6, where
(i) the radicals R¹, R² and R³ in the general formula (Ia) are hydrogen, or
(ii) the radicals R¹ and R² in the general formula (Ia) are hydrogen, R³ is a substituted or unsubstituted methyl radical, and E is a radical COOR, CONHR, CONR₂ or CN, or
(iii) two of the three radicals R¹, R² and R³ in the general formula (Ia) are hydrogen, and the third radical that is not hydrogen is a further group E, and the radicals R¹ and E are optionally bonded by ring closure formation.

8. The method as claimed in any one of claims 1 to 7, where the compound (A) is selected from the group consisting of (meth)acrylates, (meth)acrylamides, acrylonitriles, maleic anhydride, maleic acid, its esters, amides and imides, itaconic acid, its esters and amides, cyanoacrylates, vinylsulfones, vinylphosphonates, vinyl ketones, nitroethylenes, α,β-unsaturated aldehydes, vinylpyridines, β-keto-acetylenes and acetylene esters.

9. The method as claimed in any one of claims 4 to 8, where the compounds (A) carry two or more radicals CR¹R²=CR³COO.

10. The method as claimed in any one of claims 1 to 9, where the compounds (A) are selected from the group consisting of hexanediol diacrylate, dipropylene glycol diacrylate, tripropylene glycol diacrylate, trimethylolpropane triacrylate, pentaerythritol triacrylate, pentaerythritol tetraacrylate, dimethylolpropane tetraacrylate, polyether acrylates, polyester acrylates, epoxy acrylates or urethane acrylates.

11. The method as claimed in any one of claims 1 to 10, where the compound (B) has the general formula (II):
R⁴-D-H (II)
in which R⁴ is an aliphatic, aromatic or aliphatic-aromatic radical and D is CR'E, NR', PR' or S, where R' is hydrogen or an aliphatic, aromatic or aliphatic-aromatic radical and E is an electron-withdrawing substituent.

12. The method as claimed in any one of claims 1 to 11, where the compound (B) is selected from the group consisting of primary amines, secondary amines, thiols, phosphines and carbanion-forming compounds.

13. The method as claimed in claim 12, where the primary amines and secondary amines are polyamines or amines carrying further reactive radicals, where the reactive radicals are selected from the group consisting of hydrolyzable silyl groups or hydroxyl groups.

14. The method as claimed in any one of claims 1 to 13, where the addition reaction is catalyzed with basic or acidic catalysts, phosphines or lanthanoid compounds.

15. A method for producing coated pulverulent or fibrous solids, where, in a first step, the method as claimed in one of claims 1 to 14 is carried out and, in a subsequent step, pulverulent or fibrous solids are coated with the reaction product of the method carried out in the first step.

16. A method for producing coatings or plastics, where, in a first step, the method as claimed in any one of claims 1 to 14 is carried out, and, in a subsequent step, the reaction product of the method carried out in the first step is incorporated into coating material compositions or plastics.

17. A method for producing coated material surfaces, where, in a first step, the method as claimed in any one of claims 1 to 14 is carried out, and, in a subsequent step, the material surface is coated with the reaction product of the method carried out in the first step.

## Revendications

1. Procédé pour la fabrication en continue des produits de réaction par réaction d'addition, dans lequel au moins un composé (B) présentant au moins un groupement fonctionnel nucléophile est additionné à au moins un composé (A) présentant au moins une liaison multiple carbone-carbone alkènique activée ou alkinique activée, l'activation de la liaison multiple carbone-carbone s'effectuant par un substituant attirant les électrons qui est adjacent à la double liaison carbone-carbone ou la triple liaison carbone-carbone, **caractérisé en ce que** la réaction s'effectue dans une pompe-mélangeuse réactionnelle.

2. Procédé selon la revendication 1, **caractérisé en ce que** la pompe-mélangeuse réactionnelle est du type pompe tourbillonnaire, et est munie
(a) d'une chambre de mélange à symétrie de révolution avec une paroi circonférentielle et deux faces frontales qui présentent des canaux annulaires fluidiquement liés l'un avec l'autre,
(b) d'au moins une ouverture d'admission dans la chambre de mélange par laquelle le(s) composé(s) (A) est/sont introduit(s),
(c) d'au moins une ouverture d'admission dans la chambre de mélange par laquelle le(s) composé(s) (B) est/sont introduit(s),
(d) d'un rotor de mélange à entraînement magnétique dans la chambre de mélange, le rotor présentant des chanfreins disposés de manière symétrique sur la face frontale, les chanfreins formant des cellules de pression avec les canaux annulaires sur les faces frontales de la chambre de mélange, et les cellules de pression étant liées l'une avec l'autre par des alésages traversants dans le rotor de mélange, et
(e) d'une ouverture d'évacuation dans la chambre de mélange, par laquelle le mélange de réaction et/ou le produit est évacué de la pompe-mélangeuse réactionnelle,
(f) d'un circuit de thermorégulation qui peut être thermorégulé par un groupe de chauffage et/ou refroidissement externe.

3. Procédé selon la revendication 2, dans lequel la pompe-mélangeuse réactionnelle est munie d'une ouverture d'admission pour liquides de lavage.

4. Procédé selon l'une quelconque des revendications 1 à 3, **caractérisé en ce que** le composé (A) présente une des formules générales (la) et (Ib) : 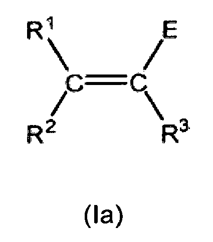 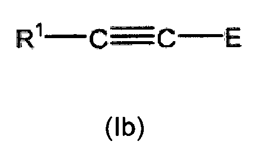 dans lesquelles
E représente un substituant attirant les électrons et R¹, R² et R³ représentent, indépendamment l'un de l'autre, H, E, ou un groupement aliphatique, aromatique ou aliphatique-aromatique, et dans lesquelles R¹ et E peuvent être liés l'un avec l'autre par cyclisation.

5. Procédé selon la revendication 4, dans lequel E est choisi dans le groupe consistant en les groupements COR, COOR, CONHR, CONR₂, CN, PO(OR)₂, pyridyle, SOR, SOOR, F ou NO₂, les groupements R indépendamment l'un de l'autre représentant H ou un groupement aliphatique, aromatique ou aliphatique-aromatique.

6. Procédé selon la revendication 4 ou 5, dans lequel au moins deux des groupements R¹, R² et R³ du composé de la formule générale (la) représentent hydrogène.

7. Procédé selon l'une quelconque des revendications 4 à 6, dans lequel
(i) les groupements R¹, R² et R³ dans la formule générale (la) représentent hydrogène, ou
(ii) les groupements R¹ et R² dans la formule générale (la) représentent hydrogène, R³ représente un groupement méthyle substitué ou non substitué et E représente un groupement COOR, CONHR, CONR₂ ou CN, ou
(iii) deux des trois groupements R¹, R² et R³ dans la formule générale (la) représentent hydrogène, et le groupement troisième qui ne représente pas de hydrogène représente un autre groupement E, et les groupements R¹ et E sont, le cas échéant, liés par cyclisation.

8. Procédé selon l'une quelconque des revendications 1 à 7, dans lequel le composé (A) est choisi dans le groupe consistant en (méth)acrylates, (méth)acrylamides, acrylonitriles, anhydride maléique, acide maléique, les esters, amides et imides de celui, acide itaconique, les esters et amides de celui, cyanoacrylates, vinylsulfones, vinylphosphonates, vinylcétones, nitro-éthylènes, aldéhydes α,β-insaturés, vinylpyridines, β-cétoacétylènes et esters d'acétylène.

9. Procédé selon l'une quelconque des revendications 4 à 8, dans lequel les composés (A) présentent deux ou plusieurs groupements CR¹R²=CR³COO.

10. Procédé selon l'une quelconque des revendications 1 à 9, dans lequel les composés (A) sont choisis dans le groupe consistant en diacrylate d'hexanediol, diacrylate de dipropylèneglycol, diacrylate de tripropylèneglycol, triacrylate de triméthylolpropane, triacrylate de pentaérythritol, tétraacrylate de pentaérythritol, tétraacrylate de diméthylolpropane, acrylates de polyéther, acrylates de polyester, acrylates d'époxyde ou acrylates d'uréthane.

11. Procédé selon l'une quelconque des revendications 1 à 10, dans lequel le composé (B) présente la formule générale (II) :
R⁴-D-H (II)
dans laquelle R⁴ représente un groupement aliphatique, aromatique ou aliphatique-aromatique, et D représente CR'E, NR', PR' ou S, R' représentant hydrogène ou un groupement aliphatique, aromatique ou aliphatique-aromatique, et E représentant un substituant attirant les électrons.

12. Procédé selon l'une quelconque des revendications 1 à 11, dans lequel le composé (B) est choisi dans le groupe consistant en amines primaires, amines secondaires, thioles, phosphines et composés formant des carbanions.

13. Procédé selon la revendication 12, dans lequel les amines primaires ou secondaires sont des polyamines ou des amines présentant des autres groupements réactifs, les groupes réactifs étant choisis dans le groupe consistant en groupes silyle hydrolysables ou groupes hydroxyle.

14. Procédé selon l'une quelconque des revendications 1 à 13, dans lequel la réaction d'addition est catalysée avec des catalyseurs basiques et acidiques, des phosphines ou des composés lanthanides.

15. Procédé pour la fabrication des matières solides pulvérulentes et/ou fibreuses revêtues, dans lequel, dans une première étape, le procédé selon l'une quelconque des revendications 1 à 14 est effectué, et, dans une étape suivante, des matières solides pulvérulentes et/ou fibreuses sont revêtues avec le produit de réaction du procédé effectué dans la première étape.

16. Procédé pour la fabrication d'agents de revêtement ou matières plastiques, dans lequel, dans une première étape, le procédé selon l'une quelconque des revendications 1 à 14 est effectué, et, dans une étape suivante, le produit de réaction du procédé effectué dans la première étape est incorporé dans des compositions de revêtement ou des matières plastiques.

17. Procédé pour la fabrication des surfaces de matériaux, dans lequel, dans une première étape, le procédé selon l'une quelconque des revendications 1 à 14 est effectué, et, dans une étape suivante, la surface de matériau est revêtue avec le produit de réaction du procédé effectué dans la première étape.
